(19) Europäisches Patentamt
European Patent Office
Office européen des brevets

(11) Veröffentlichungsnummer: **0 471 345 B1**

(12) **EUROPÄISCHE PATENTSCHRIFT**

(45) Veröffentlichungstag der Patentschrift: **02.08.95**

(21) Anmeldenummer: **91113587.9**

(22) Anmeldetag: **13.08.91**

(51) Int. Cl.[6]: **C07K 16/18**, C12P 21/08, G01N 33/531, G01N 33/577, G01N 33/74

# (54) Bestimmung von biogenen Aminen.

(30) Priorität: **14.08.90 DE 4025726**

(43) Veröffentlichungstag der Anmeldung: **19.02.92 Patentblatt 92/08**

(45) Bekanntmachung des Hinweises auf die Patenterteilung: **02.08.95 Patentblatt 95/31**

(84) Benannte Vertragsstaaten: **AT BE CH DE DK ES FR GB GR IT LI LU NL SE**

(56) Entgegenhaltungen:
**EP-A- 0 149 405**
**EP-A- 0 161 195**
**EP-A- 0 192 565**
**WO-A-86/04331**

**CHEMICAL ABSTRACTS, Band 103, Nr. 11, 16. September 1985, Columbus, Ohio, USA; K. FLURKEY et al. "Preparation and characterization of antisera and monoclonal antibodies to serotoninergic and dopaminergic ligands" Seite 471,linke Spalte, Zusammenfassung-Nr. 86 192u**

(73) Patentinhaber: **BOEHRINGER MANNHEIM GMBH**

**D-68298 Mannheim (DE)**

(72) Erfinder: **Huber, Erasmus, Dr.**
**St. Willibald 10**
**W-8911 Unterfinning (DE)**
Erfinder: **Stahl, Peter, Dr.**
**Hirtenstrasse 12**
**W-8139 Bernried (DE)**
Erfinder: **Batz, Hans-Georg, Dr.**
**Traubinger Strasse 63**
**W-8132 Tutzing (DE)**
Erfinder: **Hübner-Parajsz, Christa, Dr.**
**Marienstrasse 11**
**W-8132 Tutzing (DE)**
Erfinder: **Jungfer, Barbara**
**Beringerweg 10**
**W-8132 Tutzing (DE)**
Erfinder: **Klein, Christian, Dr.**
**Blütenstrasse 16**
**W-8120 Weilheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

## Beschreibung

Die vorliegende Erfindung betrifft ein Verfahren zur Gewinnung eines monoklonalen Antikörpers gegen ein primäres oder sekundäres biogenes Amin, wobei der Antikörper gegen ein Kopplungsprodukt aus dem biogenen Amin und einem Kopplungsreagenz gerichtet ist, sowie ein Verfahren zur immunologischen Bestimmung eines primären oder sekundären Amins in einer Probelösung.

Das erfindungsgemäße Verfahren ist geeignet zur Bestimmung von biologisch relevanten Substanzen, die primäre oder sekundäre Aminoalkylfunktionen, Aminoalkylarylfunktionen oder Guanidinofunktionen tragen. Derartige Verbindungen werden im weiteren als biogene Amine bezeichnet. Besonders geeignet ist das Verfahren zur Bestimmung derartiger biogener Amine mit einem Molekulargewicht unter 200 Dalton.

Beispiele für biogene Amine im Sinne der vorliegenden Erfindung sind Catecholamine, insbesondere Adrenalin, Noradrenalin, Dopamin sowie Spermidin, Spermin, Cadaverin, Tryptamin, Tyramin, Serotonin, Creatinin und Histamin.

Catecholamine sind Überträgerstoffe des sympatischen Nervensystems (Neurotransmitter). Sie werden in vivo über Dopa aus Tyrosin synthetisiert und durch Methylierung an der 3-Hydroxygruppe mit anschließender oxidativer Desaminierung metabolisiert.

Ihre klinische Relevanz ergibt sich aus der Diagnostik von Catecholamin produzierenden Tumoren und kardiovaskulären Abnormitäten. Die in Körperflüssigkeiten vorkommenden Konzentrationen sind durchwegs sehr niedrig.

So sind die Serumkonzentrationen für
Adrenalin ungefähr 5,9 - 35 x $10^{-10}$ mol $1^{-1}$, für
Noradrenalin ungefähr 0,5 - 4,0 x $10^{-10}$ mol $1^{-1}$ und für
Dopamin ungefähr 0,4 - 6,0 x $10^{-10}$ mol $1^{-1}$.

Das biogene Amin Histamin ist eine Mediatorsubstanz, die bei ihrer Freisetzung im Rahmen einer allergischen Reaktion eine Vielzahl unterschiedlicher Reaktionen auslöst, unter anderem Bronchokonstriktion, Kontraktion der glatten Muskulatur, Permeabilitätserhöhung des Gewebes und damit verbunden Entstehung eines Ödems sowie Dilatation der kleinen Gefäße (Flushphänomen) und Juckreiz. Im Intestinaltrakt führt es zur Erhöhung der Magensäuresekretion und Darmkontraktion.

Die physiologische Konzentration von Histamin liegt in den folgenden Bereichen:

| | |
|---|---|
| Vollblut | 20 - 100 ng/ml |
| Plasma | 0,1 - 1 ng/ml. |

Die Bestimmung von Histamin in Plasma und Serum wird hauptsächlich angewendet zur Diagnose und Verlaufskontrolle bei zahlreichen allergischen und pseudo-allergischen Krankheiten, zur weiteren Aufklärung deren Entstehungsmechanismen und Erforschung der vielfältigen Beziehungen der Mediatoren untereinander.

Die bisher verwendeten Bestimmungsmethoden für biogene Amine stützen sich im wesentlichen auf chromatographische Methoden (HPLC) mit massenspektrometrischer, elektrochemischer oder fluorometrischer Detektion, wobei letztere nicht für Plasma geeignet ist. Bisherige Bestimmungsmethoden (Antoanalyser, Massenspektrometrie, HPLC, Radioenzymatische Methoden) sind jedoch sehr arbeits- und zeitaufwendig und haben eine unzureichende Spezifität und Sensibilität. Ein weiteres sehr aufwendiges radioenzymatisches Verfahren läuft über eine $^3$H-Methylierung der 3-Hydroxygruppe (Histamintest von Pharmacia, Uppsala, Schweden) und dauert 16 bis 20 Stunden.

Für die Bestimmung von biogenen Aminen würden sich besonders immunologische Testverfahren, bei denen das Hormon als Antigen mit einem dagegengerichteten Antikörper bestimmt wird, eignen.

Die Entwicklung eines in der Praxis verwendbaren Immunoassays für biogene Amine scheiterte jedoch bisher immer an der nicht ausreichenden Spezifität der erzeugten Antikörper sowie an einer zu geringen Affinität dieser Antikörper, was eine zu geringe Empfindlichkeit des Tests zur Folge hat.

So ergaben Experimente von Spector et al. (Frontiers in Catecholamine Research, herausgegeben von E. Usdin und S.H. Snyder, Pergamon Press, New York (1973), 345-349), bei denen ein über seine Aminofunktion an Carboxylgruppen eines polymeren Trägers gekoppeltes Dopamin als Immunogen verwendet wurde, zwar Antikörper, deren Spezifität jedoch gering und deren Kreuzreaktion zu anderen Catecholaminen sehr hoch war.

Auch Geffard et al. (Neurochem. Int. 7 (1985), 403-413) koppelten Dopamin über seine Aminofunktion an ein polymeres Trägermaterial, wobei als Linker zwischen Dopamin und dem Träger Glutardialdehyd verwendet wurde. Die auf diese Weise erhaltenen Dopamin-Antikörper zeigten niedrige Affinitäts-Konstanten

zu Dopamin ($6{,}7 \times 10^3$) und Noradrenalin ($6{,}4 \times 10^5$), jedoch deutlich höhere Werte zu den entsprechenden Catecholamin-Glutardialdehyd-Derivaten ($6{,}7 \times 10^7$ und $3{,}8 \times 10^7$). Dieser Effekt ist typisch für kleine Haptene, zu denen die biogenen Amine zählen, und wird als "Linkereffekt" bezeichnet.

Darunter ist zu verstehen, daß die gewonnenen Antikörper in beträchtlichem Umfang den im Immunogen verwendeten Linker zwischen dem Hapten und dem Trägermaterial und nicht das Hapten selbst erkennen. Die Affinität des Antikörpers zum Hapten-Linker-Konjugat ist oftmals um Größenordnungen höher als zum underivatisierten Hapten.

Deshalb wurde versucht, Methoden zu entwickeln, nach denen das biogene Amin in der Analytlösung mit dem Linker derivatisiert werden kann und anschließend die immunologische Bestimmung des Analyten über die Bestimmung des Derivats erfolgt.

Beispielsweise wird beschrieben, wie mit Hilfe eines N-Hydroxysuccinimidesters (EP-A 0 161 195) bzw. mit Benzochinon (EP-A 0 192 565) eine derartige Derivatisierung eines biogenen Amins und anschließende Bestimmung des Derivats durchgeführt wird. Die Derivatisierung mit N-Hydroxysuccinimidestern hat jedoch den Nachteil, daß mit ihr im wesentlichen nur primäre Amine in ausreichendem Maße erfaßt werden können. Die Reaktivität von solchen Aktivestern gegenüber sekundären Aminen (wie z.B. Adrenalin) ist im wäßrigen Medium im Verhältnis zu primären Aminen (wie z.B. Noradrenalin oder Dopamin) deutlich geringer.

Bei der Umsetzung von Aminen mit Benzochinon in Medien wie Vollblut oder Serum, die eine Vielzahl verschiedener aminogruppenhaltiger Verbindungen enthalten können, kann es weiterhin zu einer Fülle von Nebenreaktionen durch Quervernetzungen über das bifunktionelle Benzochinon kommen. Dadurch kann die effektiv gemessene Konzentration des zu bestimmenden Amins auf nicht reproduzierbare Weise verringert werden.

In der DE-A 26 08 096 sind Antikörper gegen Adrenalin beschrieben, allerdings auch mit teilweise hoher Kreuzreaktion zu Metanephrin und Synephrin.

Die Immunogensynthese erfolgte durch Kupplung über die 5-Position des Aromaten mittels Mannich-Kondensation mit Formaldehyd an die Lysinreste des Trägerproteins.

Diese Ergebnisse konnten jedoch durch andere Arbeitsgruppen (Diener et al., Clin. Chim. Acta 109 (1981), 1-11) nicht bestätigt werden. Auch die Tatsache, daß bisher kein kommerzieller Test auf Basis der DE-A 26 08 096 bekannt wurde, obwohl die Arbeiten schon mehr als 10 Jahre zurückliegen, spricht nicht für eine praktische Verwertbarkeit der Ergebnisse.

Es ist also bisher nicht gelungen, über eine konventionelle Immunogensynthese zu Antikörpern gegen primäre und sekundäre biogene Amine zu kommen, mit deren Hilfe ein hinreichender sensitiver immunologischer Test ohne störende Kreuzreaktivitäten konzipierbar wäre. Die Konzentrationen der Parameter sind einerseits zu gering, andererseits ist die Anzahl der kreuzreagierenden Metaboliten zu hoch, als daß einer der in der Literatur verfolgten Ansätze eine vernünftige Erfolgsaussicht gewähren würde.

Aufgabe der vorliegenden Erfindung war es somit, ein Verfahren zur Bestimmung niedermolekularer primärer oder sekundärer Amine (biogene Amine), insbesondere mit einem Molekulargewicht $< 200$, wie etwa Catecholamine zu entwickeln. Die Methode sollte prinzipiell auf alle niedermolekularen Haptene mit primären und sekundären Aminofunktionen anwendbar sein, gegen die bisher keine Antikörper mit ausreichender Affinität erhalten werden konnten.

Die erfindungsgemäße Aufgabe wird durch ein Verfahren zur Gewinnung eines monoklonalen Antikörpers gegen ein primäres oder sekundäres biogenes Amin gelöst, wobei der Antikörper gegen ein Kopplungsprodukt aus dem biogenen Amin und einem Kopplungsreagenz gerichtet ist, wobei man

(1) ein primäres oder sekundäres biogenes Amin über seine Aminofunktion an ein Kopplungsreagenz bindet, welches die allgemeine chemische Struktur besitzt:

$$X=C=N\text{-}R\text{-}Y$$

worin

X für O oder S steht,

R eine nicht substituierte oder substituierte aromatische, heteroaromatische oder Cycloalkylgruppe ist,

Y für eine Carboxylgruppe mit der allgemeinen Formel

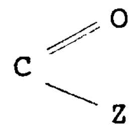

steht, worin Z (a) OH oder $O^-$ oder (b) ein anderer unter den Bedingungen der Kopplungsreaktion nicht mit einer Aminofunktion reagierender Rest $Z^1$ ist, der jedoch durch einen oder mehrere Reaktionsschritte in einen reaktiven Ester-, Säurechlorid- oder Säureanhydridrest überführbar ist,

(2) den Rest Z der Carboxylgruppe des in Schritt (1) entstehenden Kopplungsprodukts in einen reaktiven Ester-, Säurechlorid- oder Säureanhydridrest überführt,

(3) das aktivierte Kopplungsprodukt aus Schritt (2) an ein Trägermaterial bindet, so daß man ein Träger-gebundenes Kopplungsprodukt erhält, das als Immunogen dient,

(4) ein Versuchstier mit dem Immunogen aus Schritt (3) immunisiert, und

(5) auf an sich bekannte Weise monoklonale Antikörper gegen das Kopplungsprodukt aus dem biogenen Amin und dem Kopplungsreagenz aus dem immunisierten Versuchstier gewinnt.

Der nach dem erfindungsgemäßen Verfahren gewonnene monoklonale Antikörper ist gegen ein Kopplungsprodukt aus dem primären oder sekundären biogenen Amin und einem Kopplungsreagenz gerichtet. Dabei wird die Aminofunktion des biogenen Amins durch Umsetzung mit Isocyanaten bzw. Isothiocyanaten zu Harnstoffen bzw. Thioharnstoffen schnell und ohne Nebenreaktionen derivatisiert, wobei ein größeres Hapten entsteht, das besser zur Immunisierung als das primäre oder sekundäre Amin geeignet ist.

Das Kopplungsreagenz besitzt die allgemeine chemische Struktur:

X = C = N-R-Y.

X bedeutet O oder S, vorzugsweise S. R bedeutet eine nicht substituierte oder substituierte aromatische, heteroaromatische oder Cycloalkylgruppe, worin R vorzugsweise ein Phenyl-, Nitrophenyl-, Dinitrophenyl-, Naphthyl-, Benzyl-, Xylyl-, Pyridyl- oder Cyclohexylrest ist. Besonders bevorzugt ist R ein Phenylrest.

In einer besonders bevorzugten Ausführungsform der vorliegenden Erfindung verwendet man als Kopplungsreagenz 3-Isothiocyanatobenzoesäure (ITCB) oder ein Salz oder ein Derivat davon. ITCB läßt sich relativ leicht auf bekannte Weise aus 3-Amino-benzoesäure und Thiophosgen gewinnen.

Ist das Kopplungsreagenz ein Isocyanat, so ist es auf einfache Weise durch Umsetzung des entsprechenden primären Amins mit Phosgen erhältich. Ist das Kopplungsreagenz ein Isothiocyanat, so ist es durch Umsetzung des entsprechenden primären Amins mit Thiosphosgen erhältlich. Eine andere Möglichkeit zur Herstellung von Isothiocyanaten ist, wenn man das entsprechende primäre Amin mit Schwefelkohlenstoff in Gegenwart von Natronlauge umsetzt. Das sich hierbei bildende Na-Salz der N-substituierten Dithiocarbamidsäure reagiert mit Chlorameisensäureethylesterzu COS, Ethanol und dem Isothiocyanat.

Y steht für eine Carboxylgruppe mit der allgemeinen Formel:

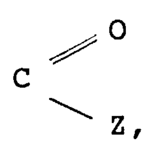

worin Z (a) OH oder $O^-$ oder (b) ein anderer unter den Bedingungen der Kopplungsreaktion nicht mit einer Aminofunktion reagierender Rest $Z^1$ ist, der jedoch durch einen oder mehrere Reaktionsschritte in einen reaktiven Ester-, Säurechlorid- oder Säureanhydridrest überführbar ist.

Z kann beispielsweise OH oder $O^-$ bedeuten, so daß die Carboxylgruppe Y eine Carbonsäure- oder Carboxylatgruppe ist. Eine Carbonsäure- oder Carboxylatgruppe reagiert unter den Bedingungen der Kopplungsreaktion, d.h. der Harnstoff- oder Thioharnstoffbildung nicht mit einer Aminogruppe, wie dem Fachmann bekannt ist.

Um für das Kopplungsreagenz eine bessere Löslichkeit während der Derivatisierungsreaktion mit der Aminofunktion des biogenen Amins zu erreichen, kann die Carboxylgruppe Y auch mit einem Rest $Z^1$ substituiert sein. $Z^1$ bedeutet demnach günstigerweise eine hydrophile Gruppe, welche die Löslichkeit des

Kopplungsreagenz in wäßrigem Medium erhöht. Z kann vorzugsweise $OR^1$ oder $NR^2R^3$ bedeuten, wobei $R^1$ der Rest eines Alkohols $R^1OH$ ist, der neben seiner Hydroxylfunktion mindestens eine weitere hydrophile Gruppe enthält, und worin mindestens einer von $R^2$ und $R^3$ ein Rest einer Aminoverbindung $HNR^2R^3$ ist, die neben der Aminogruppe noch mindestens eine weitere hydrophile Gruppe enthält, und der andere Wasserstoff oder $C_1$-$C_3$-Alkyl sein kann.

$R^1$, $R^2$ und $R^3$ können Reste sein, die mindestens noch eine weitere hydrophile Gruppe enthalten. Unter dem Begriff "hydrophile Gruppe" ist in diesem Sinne eine Hydroxy-, Ether-, Carbonsäure-, Carbonsäureamid-, Acetal-, Ketal- oder eine Aminogruppe zu verstehen. Die Reste $R^1$, $R^2$ und $R^3$ können auch mehrere gleiche oder/und verschiedene hydrophile Gruppen enthalten.

Beispiele für geeignete Alkohole $R^1OH$ sind Ethylenglykol, Propylenglykol, Diethylenglykol, Triethylenglykol, Ethylenglykolmonomethylether, Ethylenglykolmonoethylether, Diethylenglykolmonomethylether, Triethylenglykolmonomethylether und dergleichen. Vorzugsweise ist der Alkohol $R^1OH$ Ethylenglykol, Diethylenglykol oder Triethylenglykol.

Die Aminoverbindung $R^2R^3NH$ kann z.B. Ethanolamin, Aminopropanol, Aminopropandiol, Aminobutanol, Aminobutandiol, Diethanolamin, Methoxyethylamin, Aminoethoxyethanol, Morpholin, 4-Aminomorpholin, 3-Morpholino-1-ethylamin, 3-Morpholino-1-propylamin, Glycin, Serin, Alanin, Glycylglycin, Glucosamin, Fructosamin, Galactosamin oder dergleichen sein. Vorzugsweise ist die Aminoverbindung $R^2R^3NH$ Ethanolamin oder Glucosamin.

Mit einem Rest $Z^1$ substituierte Kopplungsreagenzien sind z.B. erhältlich, indem man anstelle der entsprechenden Aminocarbonsäure $H_2N$-R-$CO_2$ H die substituierte Aminocarbonsäure $H_2N$-R-$COZ^1$ mit Phosgen bzw. Thiophosgen umsetzt.

Verwendet man zur Derivatisierungsreaktion mit dem biogenen Amin ein mit einer hydrophilen Gruppe verknüpftes Kopplungsreagenz, so kann es erforderlich sein, nach der Derivatisierungsreaktion den vorzugsweise hydrophilen Rest $Z^1$ abzuspalten, um ein reaktives Kopplungsprodukt herzustellen. Die Hydrolyse der Säureamid- bzw. Esterbindung kann dabei auf eine übliche, dem Fachmann bekannte Weise geschehen, wobei jedoch zu beachten ist, daß man solche Reaktionsbedingungen wählt, welche die Harnstoff- bzw. Thioharnstoff-Bindung zwischen biogenem Amin und Kopplungsreagenz nicht zerstören.

Die Hydrolyse kann im Falle einer Esterbindung beispielsweise durch mehrstündiges Rühren der Verbindung in methanolischer Kalilauge oder im Fall einer Amidbindung beispielsweise duch mehrstündiges Rühren in 1 N Salzsäure erfolgen.

Enthält der Rest $Z^1$ eine freie Carbonsäuregruppe (z.B. bei Verwendung von Glycin oder anderen Aminosäuren als Verbindung $R^2R^3NH$) so ist eine Abspaltung von $Z^1$ nicht zwingend notwendig, da die freie Carbonsäuregruppe direkt (wie unten beschrieben) in einen reaktiven Rest überführt werden kann.

Nach Entfernung der vorzugsweise hydrophilen Gruppe $Z^1$ liegt der Rest Z des Kopplungsreagenz als Carbonsäure oder Carboxylatgruppe vor, die anschließend in einen reaktiven Ester-, Säurechlorid- oder Säureanhydridrest überführt wird. Dies kann durch alle üblichen, dem Fachmann bekannte Verfahren geschehen. So kann man z.B. günstigerweise eine Carbonsäuregruppe durch Umsetzung mit Thionylchlorid in einen reaktiven Säurechloridrest überführen.

Vorzugsweise überführt man die Carbonsäuregruppe Y des Kopplungsprodukts durch Umsetzung mit N-Hydroxysuccinimid und Dicyclohexylcarbodiimid in einen reaktiven N-Hydroxysuccinimidesterrest. Dies kann z.B. durch Zugabe annähernd äquimolarer Mengen der Reaktionskomponenten in Dioxan und mehrstündiges Rühren bei 20 °C geschehen.

Anschließend wird das aktivierte Kopplungsprodukt an ein Trägermaterial gebunden, so daß man ein trägergebundenes Kopplungsprodukt erhält, das als Immunogen dient. Als Trägermaterial kann man alle für diesen Zweck dem Fachmann bekannten Materialien verwenden, z.B. Proteine wie ein Immunglobulin oder Rinderserumalbumin oder auch ein festes Trägermaterial. Vorzugsweise verwendet man als Trägermaterial ein Protein. Besonders bevorzugt verwendet man als Trägermaterial $\beta$-Galactosidase oder ein Fragment davon.

Der nächste Schritt ist die Herstellung von Antikörpern. Diese erfolgt durch die Immunisierung eines Versuchstiers mit dem Immunogen. Dieser Schritt des erfindungsgemäßen Verfahrens kann auf eine übliche, dem Fachmann bekannte Weise geschehen. Vorzugsweise verabreicht man das Immunogen dem Versuchstier in Kombination mit einem Adjuvans. Besonders bevorzugt verwendet man als Adjuvans Freundsches Adjuvans oder Aluminiumhydroxid zusammen mit Bortadella pertussis. Die Immunisierung erfolgt vorzugsweise über mehrere Monate mit mindestens vier Immunisierungen in vier- bis sechswöchigem Abstand. Die Injektion des Immunogens erfolgt vorzugsweise intraperitoneal.

Aus so immunisierten Tieren werden B-Lymphozyten gewonnen, die mit einer permanenten Myelomzellinie fusioniert werden. Die Fusionierung erfolgt nach dem bekannten Verfahren von Köhler und Milstein (Nature 256, 1975, 495-497). Die hierbei gebildeten Primärkulturen von Hybridzellen werden in üblicher

Weise, z.B. unter Verwendung eines handelsüblichen Zellsorters oder durch "limited dilution" kloniert. Es werden jeweils die Kulturen weiterverarbeitet, die in einem geeigneten Testverfahren, beispielsweise einem Enzym-Immuno-Assay (ELISA-Verfahren), positiv gegen das Kopplungsprodukt aus biogenem Amin und Kopplungsreagenz (z.B. Histamin-ITCB) und negativ bzw. nur wenig mit anderen verwandten Serumbestandteilen (z.B. Histidin, Lysin, Methylhistamin, Acetylhistamin etc.) und deren ITCB-Derivaten reagieren. Man erhält so mehrere Hybridoma-Zellinien, die die erfindungsgemäßen monoklonalen Antikörper produzieren. Nach bekannten Methoden können diese Zellinien kultiviert und die von ihnen produzierten monoklonalen Antikörper isoliert werden.

Als biogenes Amin für das erfindungsgemäße Verfahren verwendet man vorzugsweise Histamin oder ein Catecholamin, ausgewählt aus Adrenalin, Noradrenalin und Dopamin. Bei der Umsetzung des biogenen Amins mit dem Kopplungsreagenz ist die Reaktionsrate auch in wäßrigem Medium so hoch, daß innerhalb von Minuten eine annähernd vollständige Umsetzung erzielt wird. Die Reaktion verläuft einheitlich und führt zu stabilen Produkten (Harnstoffen bzw. Thioharnstoffen). Die Reaktion verläuft sowohl bei primären Aminen wie Dopamin und Histamin, als auch bei sekundären Aminen wie Adrenalin glatt.

Ein weiterer Gegenstand der vorliegenden Erfindung ist ein Kopplungsprodukt aus einem primären oder sekundären biogenen Amin $HNR^4R^5$ und einem Kopplungsreagenz, wobei das Kopplungsprodukt folgende allgemeine Struktur besitzt:

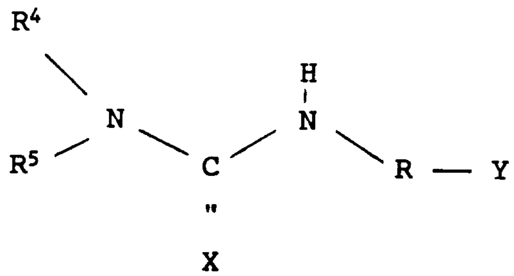

worin $R^4$ und $R^5$ die dem jeweiligen biogenen Amin entsprechenden Reste bedeuten, und
X für O oder S steht,
R eine nicht substituierte oder substituierte aromatische, heteroaromatische oder Cycloalkylgruppe ist,
Y für eine Carboxylgruppe mit der allgemeinen Formel

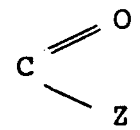

steht, worin Z (a) OH oder $O^-$ oder (b) ein anderer unter den Bedingungen der Kopplungsreaktion nicht mit einer Aminofunktion reagierender Rest $Z^1$ ist, der jedoch in einen reaktiven Ester-, Säurechlorid- oder Säureanhydridrest überführbar ist.

Vorzugsweise ist das biogene Amin $HNR^4R^5$ Histamin oder ein Catecholamin, ausgewählt aus Adrenalin, Noradrenalin und Dopamin. Vorzugsweise ist das Kopplungsreagenz Isothiocyanatobenzoesäure (ITCB), ein Salz oder Derivat davon.

Durch Derivatisierung des als Hapten verwendeten biogenen Amins mit ITCB konnte ein neues immunologisches Verfahren und Reagenz bereitgestellt werden, mit dessen Hilfe biogene Amine auch in physiologisch sehr geringen Konzentrationen (0 bis 1 ng/ml) in Gegenwart einer großen Zahl an kreuzreagierenden Substanzen im Blut spezifisch erfaßt werden können. Gelöst wird diese Aufgabe durch das erfindungsgemäße immunologische Verfahren und Reagenz, bei dem mindestens ein monoklonaler Antikörper eingesetzt wird, der spezifisch gegen das Kopplungsprodukt aus biogenem Amin und Kopplungsreagenz gerichtet ist und mit kritischen Substanzen im Serum weniger als 0,1% kreuzreagiert.

Das Wesentliche der vorliegenden Erfindung ist darin zu sehen, daß es überraschenderweise gelang, durch die Derivatisierung der biogenen Amine mit einem Kopplungsreagenz und nachfolgende Immunisierung mit dem Kopplungsprodukt monoklonale Antikörper bereitzustellen, die spezifisch gegen das Kopplungsprodukt gerichtet sind und die daher eine spezifische Bestimmung des jeweiligen derivatisierten Amins ermöglichen.

Ein weiterer Gegenstand der Erfindung sind daher monoklonale Antikörper gegen ein erfindungsgemäßes Kopplungsprodukt aus biogenem Amin und einem (Iso(thio)cyanat-Kopplungsreagenz (z.B. Histamin-ITCB).

Durch das erfindungsgemäße Verfahren ist es möglich, monoklonale Antikörper zu erhalten, die eine Kreuzreaktivität mit kritischen Substanzen von weniger als 0,1 % besitzen. Als kritische Substanzen in diesem Sinne sind andere biogene Amine mit ähnlicher Struktur, sowie deren Kopplungsprodukte zu verstehen.

Die erfindungsgemäßen monoklonalen Antikörper zeichnen sich vorzugsweise auch dadurch aus, daß sie eine sehr hohe Affinität gegen ein erfindungsgemäßes Kopplungsprodukt besitzen. Besonders bevorzugte monoklonale Antikörper weisen eine Kreuzreaktivität gegenüber anderen primären und sekundären Aminen, wie Histidin, Lysin, Serotonin und deren Kopplungsprodukten (z.B. ITCB-Derivaten) von weniger als 0,01 % auf. Zur Bestimmung der Relativen Affinität und der Kreuzreaktivität mit den kritischen Substanzen stehen die üblichen, dem Fachmann bekannten Verfahren zur Verfügung.

Die erfindungsgemäßen monoklonalen Antikörper eignen sich daher hervorragend dazu, in einer Probe, beispielsweise Serum oder Plasma ein Kopplungsprodukt (z.B. Histamin-ITCB) in Gegenwart anderer strukturähnlicher Substanzen spezifisch zu bestimmen. Für diese Bestimmungsverfahren können die monoklonalen Antikörper als solche oder Fragmente hiervon, welche die entsprechenden immunologischen Eigenschaften aufweisen (Fab-Fragmente), verwendet werden. Unter dem Begriff "monoklonale Antikörper" werden daher sowohl die vollständigen Antikörper als auch die Fragmente verstanden.

Beispiele für erfindungsgemäße monoklonale Antikörper sind Antikörper, die gegen ein Kopplungsprodukt aus Histamin und Isothiocyanatobenzoesäure gerichtet sind, und die von den Hybridoma-Zellinien 1.47.74 (ECACC 90071902) bzw. 1.17.44 (ECACC 90071901) produziert werden.

Weiterhin ein Gegenstand der vorliegenden Erfindung ist somit ein Verfahren zur Bestimmung eines primären oder sekundären biogenen Amins in einer Probelösung, wobei man

(1) das biogene Amin in der Probelösung über seine Aminofunktion an ein Kopplungsreagenz bindet, welches die allgemeine chemische Struktur besitzt:

$$X=C=N\text{-}R\text{-}Y$$

worin X für O oder S steht,
R eine nicht substituierte oder substituierte aromatische, heteroaromatische oder Cycloalkylgruppe ist,
Y für eine Carboxylgruppe mit der allgemeinen Formel

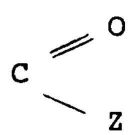

steht, worin Z (a) OH oder $O^-$ oder (b) ein anderer unter den Bedingungen der Kopplungsreaktion nicht mit einer Aminofunktion reagierender Rest $Z^1$ ist, der jedoch in einen reaktiven Ester-, Säurechlorid- oder Säureanhydridrest überführbar ist,

(2) die Probelösung mit einem oder mehreren monoklonalen Antikörpern gegen das in Schritt (1) entstehende Kopplungsprodukt aus biogenem Amin und Kopplungsreagenz inkubiert,

(3) durch Quantifizierung der Antikörper/Antigen-Reaktion auf übliche Weise die Konzentration des biogenen Amins in der Probe bestimmt.

Vorzugsweise verwendet man als Kopplungsreagenz Isothiocyanatobenzoesäure, ein Salz oder ein Derivat davon. Bevorzugt ist das zu bestimmende biogene Amin Histamin oder ein Catecholamin, ausgewählt aus Adrenalin, Noradrenalin und Dopamin.

Im ersten Schritt zur Bestimmung des biogenen Amins in einer Probelösung muß durch Umsetzung mit dem Kopplungsreagenz das erfindungsgemäße Kopplungsprodukt erzeugt werden, gegen welches der erfindungsgemäße Antikörper gerichtet ist. Dazu hat es sich als günstig erwiesen, das Kopplungsreagenz im Überschuß zur Probelösung zu geben und das Gemisch bei erhöhter Temperatur, vorzugsweise von 30 bis 70 °C, besonders bevorzugt von 50 bis 60 °C zu inkubieren. Auf diese Weise entsteht in der Probelösung das erfindungsgemäße Kopplungsprodukt aus dem zu bestimmenden biogenen Amin und dem Kopplungsreagenz.

Anschließend muß das überschüssige Kopplungsreagenz in der Probelösung abgefangen werden. Dies kann vorzugsweise durch Zugabe einer geeigneten Aminoverbindung geschehen. Die hierfür "geeignete

Aminoverbindung" kann Ammoniak, ein primäres oder sekundäres Amin sein. Dabei ist darauf zu achten, daß das Additionsprodukt aus Abfang-Aminoverbindung und Kopplungsreagenz keine Kreuzreaktivität mit dem (später eingesetzten) Antikörper gegen Kopplungsreagenz und dem zu bestimmenden biogenen Amin hat. Als besonders geeignet zu diesem Zweck haben sich Ammoniak und Tris erwiesen. Die so vorbereiteten Proben können dann direkt zur Konzentrationsbestimmung des als Kopplungsprodukt derivatisiert vorliegenden biogenen Amins mit Hilfe eines erfindungsgemäßen Antikörpers eingesetzt werden.

Neben dem zu bestimmenden Kopplungsprodukt liegen oft in der Probe noch weitere derivatisierte chemisch verwandte biogene Amine vor. Bei einem Test zur Bestimmung von Histamin-ITCB sind hier z.B. insbesondere die ITCB-Kopplungsprodukte von Histidin und Lysin relevant. Überraschenderweise wurde hier eine verschwindend geringe Kreuzreaktivität von 8 untersuchten monoklonalen Histamin-ITCB-Antikörpern gegen Lysin-ITCB (0 %) und Histidin-ITCB (0 bis 0,01 %) gefunden. Daraus wird ersichtlich, daß die erfindungsgemäßen Antikörper gegen ein Kopplungsprodukt und ein bestimmtes biogenes Amin eine ausreichend hohe Spezifität besitzen, die zur Bestimmung geringster Mengen (< 1 ng/ml) des biogenen Amins in der Probelösung ausreichend ist.

Die genauen Bedingungen der immunologischen Reaktion und die Bestimmung der Konzentration des derivatisierten biogenen Amins in der Probe durch Quantifizierung der Antikörper/Antigen-Reaktion kann auf eine dem Fachmann bekannte übliche Weise geschehen. Vorzugsweise quantifiziert man die Antigen/Antikörper-Reaktion durch einen kompetitiven ELISA-Test. Es sind jedoch auch sämtliche anderen bekannten Methoden zur immunologischen Bestimmung der Antigen/Antikörper-Reaktion geeignet (siehe z.B. Oellerich, J. Clin. Chem. Clin. Biochem. 22 (1984), 895-904 oder Kage und Köttgen, MTA 3 (1988), 797-804).

Die Erfindung wird durch die folgenden Beispiele in Verbindung mit den Figuren 1 bis 3 näher erläutert: Es zeigen:

Fig. 1 die Derivatisierung und Immunogenherstellung aus Histamin mit 3-Isothiocyanatobenzoesäure als Kopplungsreagenz,

Fig. 2 die Derivatisierung und Immunogenherstellung aus Dopamin und Adrenalin mit 3-Isothiocyanatobenzoesäure als Kopplungsreagenz und

Fig. 3 die Derivatisierung und Immunogenherstellung aus Adrenalin mit 5-Isothiocyanato-2-nitrobenzoesäure als Kopplungsreagenz.

Beispiel 1

Herstellung von ITCB derivatisiertem Histamin (Immunogensynthese)

Das Prinzip der Immunogenherstellung ist schematisch in Fig. 1 dargestellt und beruht auf der Derivatisierung des Histamins 2 mit 3-Isothiocyanato-benzoesäure 1 (ITCB). ITCB reagiert mit primären und sekundären Aminen wie Histamin in wässriger Lösung innerhalb von Minuten nahezu vollständig zum entsprechenden Thioharnstoff-Derivat 3. Dieses Derivat bzw. Kopplungsprodukt wird isoliert und zum Hydroxy-Succinimid-Ester 4 umgesetzt. Das so gewonnene aktivierte Hapten wird an $\beta$-Galactosidase als Trägerprotein gekoppelt.

**a) N-(3-Carboxyphenyl)-N'-[2-(4-imidazolyl)ethyl]thioharnstoff (Histamin-ITCB) 3**

1.79 g (10 mmol) 3-Isothiocyanatobenzoesäure **1** und 1.11 g (10 mmol) Histamin **2** werden 150 ml THF/Wasser 2/1 (v/v) gelöst und 24 h bei 20 °C gerührt. Anschließend dampft man die Lösung am Wasserstrahlvakuum ein, löst den Rückstand in ca 20 ml Methanol und reinigt das Rohprodukt durch Chromatographie an einer Kieselgelsäule (8.5x30cm). Als Eluent verwendet man Methanol/Essigester 1/1 (v/v). Die entsprechenden Fraktionen werden vereinigt, das Lösungsmittel am Rotationsverdampfer entfernt und der Rückstand an der Hochvakuumpumpe getrocknet.

Ausbeute: 2.05 g zähes, leicht bräunliches Öl (71 % d.Th.).

DC: Kieselgel, Methanol/Essigester/Eisessig 49/49/2 (v/v/v); $R_f$ = 0.24

**b) N-[2-(4-Imidazolyl)ethyl]-N'-[3-(N-succinimidyloxycarbonylphenyl)]thioharnstoff 4 (Histamin-ITCB-NHS)**

290 mg (1 mmol) Histamin-ITCB **3** werden in 50 ml absolutem Dioxan/DMF 5/1 (v/v) gelöst und mit 138 mg (1.2 mmol) N-Hydroxysuccinimid und 247 mg (1.2 mmol) N,N'-Dicyclohexylcarbodiimid versetzt. Man läßt die Lösung 18 h bei 20 °C rühren, dann entfernt man das Lösungsmittel im Hochvakuum und nimmt

den Rückstand in 50 ml Dioxan/Methanol 5/1 (v/v) auf. Man filtriert vom ungelöst gebliebenen Harnstoff ab, dampft wiederum zur Trockne ein und löst in ca 3 ml DMF. Unter Rühren versetzt man genau solange mit Dioxan, bis eine leichte Trübung eintritt. Dann läßt man 1 h bei 4°C stehen, filtriert und fällt das Produkt **4** durch Zutropfen von ca 50 ml Diisopropylether zum Filtrat aus. Man saugt über eine Filternutsche ab und trocknet den Ester an der Hochvakuumpumpe.

Ausbeute: 165 mg weißes Pulver (43 % d.Th.).

DC: Kieselgel, Methanol/Essigester/Eisessig 49/49/2 (v/v/v); $R_f$ = 0.53

**c) Histamin-ITCB-β-Galactosidase-Immunogen 5**

100 mg Histamin-ITCB-NHS **4** werden in 5 ml DMF gelöst und zu einer Lösung von 1 g β-Galactosidase in 500 ml 0.1 N Kaliumphosphatpuffer pH 8.5 gegeben. Man läßt 20 h bei 20°C rühren, dialysiert gegen Wasser und lyphilisiert. Das fertige Immunogen wird bei -20°C gelagert und zur Immunisierung in 0.1 N Kaliumphosphatpuffer, pH 7.0, + 0.9 % NaCl gelöst.

**d) N-(3-Carboxyphenyl)-N'-[2-(3,4-dihydroxphenyl)ethyl]thioharnstoff (Dopamin-ITCB) 7a**

1.90 g (10 mmol) Dopamin-Hydrochlorid **6a** (Fig. 2) werden zusammen mit 1.79 g (10 mmol) 3-Isothiocyanatobenzoesäure **1** in 10 ml THF suspendiert und mit 1 ml Triethylamin versetzt. Man läßt 18 h bei 20°C rühren, dann gibt man die trübe Lösung auf eine Kieselgelsäule (8.5x30cm) und eluiert mit Essigester/Methanol 1/1 (v/v). Die entsprechenden Fraktionen werden vereinigt, 1 h mit 0.5 g Aktivkohle behandelt und dann filtriert. Man entfernt das Lösungsmittel im Wasserstrahlvakuum und trocknet den Rückstand an der Hochvakuumpumpe.

Ausbeute: 2.35 g leicht bräunliches, zähes Öl (71 % d. Th.)

DC: Kieselgel, Essigester/Eisessig 19/1 (v/v); $R_f$ = 0.73 .

**e) N-[2-(3,4-Dihydroxyphenyl)ethyl]-N'-[3-(N-succinimidyloxycarbonylphenyl)]thioharnstoff 8a (Dopamin-ITCB-NHS)**

332 mg (1 mmol) Dopamin-ITCB **7a** (Fig. 2) werden in 10 ml Dioxan gelöst und mit 127 mg (1.1 mmol) N-Hydroxysuccinimid und 227 mg (1.1 mmol) Dicyclohexylcarbodiimid versetzt. Man läßt die Lösung 18 h bei 20°C rühren, dann wird vom ausgefallenen Dicyclohexylharnstoff abfiltriert, das Lösungsmittel im Wasserstrahlvakuum entfernt und der Rückstand in 20 ml Essigester aufgenommen. Man filtriert erneut, engt die Lösung auf ca 5 ml ein und gibt sie über eine 5 cm dicke Kieselgelschicht (Filternutsche, Durchmesser ca 5 cm). Es wird mit 50 ml Essigester nachgewaschen und das Eluat auf etwa 10 ml eingeengt. Das Produkt **8a** (Fig. 2) fällt man unter Rühren durch Zugabe von 50 ml Diisopropylether zu der Lösung aus, saugt ab und trocknet im Exsikkator über Phosphorpentoxid.

Ausbeute: 205 mg nahezu farbloses Pulver (48 % d.Th.).

DC: Kieselgel, Essigester/Eisessig 99/1 (v/v); $R_f$ = 0.50 .

**f) Dopamin-ITCB-β-Galactosidase-Immunogen 9a**

Die Herstellung (Fig. 2) erfolgt analog Beispiel 1c).

**g) N'-(3-Carboxyphenyl)-N-[2-(3,4-dihydroxyphenyl)-2-hydroxyethyl]-N-methylharnstoff 7b (Adrenalin-ITCB)**

Die Herstellung (Fig. 2) erfolgt analog Beispiel 1d) ohne Zusatz von Triethylamin.
Eingesetzte Menge Adrenalin **6b** 1.83 g (10 mmol).

Ausbeute: 2.85 g nahezu farbloses, zähes Öl(79 % d.Th.).

DC: Kieselgel, Essigester/Eisessig 19/1 (v/v); $R_f$ = 0.62 .

**h) N-[2-(3,4-dihydroxyphenyl)-2-hydroxyethyl]-N-methyl-N'-[3-(N-succinimidyloxycarbonylphenyl)]-thioharnstoff 8b (Adrenalin-ITCB-NHS)**

Die Herstellung (Fig. 2) erfolgt analog Beispiel 1e). Eingesetzte Menge Adrenalin-ITCB **7b** 3.62 g (1 mmol).

Ausbeute: 250 mg farbloses Pulver (54 % d.Th.).
DC: Kieselgel, Essigester/Eisessig 99/1 (v/v); $R_f$ = 0.48 .

**i) Adrenalin-ITCB-β-Galactosidase-Immunogen 9b**

Die Herstellung (Fig. 2) erfolgt analog Beispiel 1c).

Beispiel 2

Gewinnung von monoklonalen Antikörpern gegen Histamin-ITCB

Immunisierung

Balb/c-Mäuse, 8-12 Wochen alt, werden mit 100 µg Histamin-ITCB-β-Galactosidase (Herstellung nach Beispiel 1c) in Freund'schem Adjuvants emulgiert, intraperitoneal immunisiert. Nach 6 Wochen werden drei weitere Immunisierungen in 4-wöchigem Abstand durchgeführt. Dabei werden jeweils 100 µg des Immunogens, in inkomplettem Freund'schem Adjuvants emulgiert, intraperitoneal verabreicht.

Fusion

Ungefähr vier Monate nach der ersten Immunisierung wird fusioniert. An den letzten drei Tagen vor der Fusion wird jeweils noch einmal mit 100 µg und zweimal mit 50 µg Histamin-ITCB-β-Galactosidase in PBS (phosphate buffered saline), intravenös immunisiert.

Zur Fusion werden in Anlehnung an Galfré, Methods in Enzymology, 73 (1981), Seite 3, $10^8$ Milzzellen einer immunisierten Maus mit 2 x $10^7$ Myelomzellen (P3x63Ag8-653, ATCC-CRL 8375) gemischt und anschließend 10 Minuten zentrifugiert (300 g, 4 °C). Die Zellen werden noch einmal mit BSS (= Balanced Salt Solution) gewaschen und bei 400 g zentrifugiert. Der Überstand wird entfernt. Das Zellsediment wird mit 1 ml PEG 50 % w/v-Lösung in PBS (MG 4000, Merck) versetzt. Danach wird bei Raumtemperatur 5 ml RPMI 1640 ohne fötales Kälberserum (FKS), anschließend noch einmal 5 ml RPMI 1640, mit 10 % FKS langsam zugetropft, mit Medium auf 50 ml aufgefüllt und 10 Minuten bei 400 g zentrifugiert. Die sedimentierten Zellen werden erneut in RPMI 1640-Medium mit 10 % FKS aufgenommen. Je 1 x $10^5$ bzw. 5 x $10^4$ Milzzellen werden auf 24 well-Zellkulturplatten (Firma Nunc) eingesät. Dem Hypoxanthin-Azaserin-Selektionsmedium (100 mmol/l Hypoxanthin, 1µg/ml Azaserin) wird 5 U/ml Interleukin 6 (IL 6) als Wachstumsfaktor zugesetzt. Nach ca. 7-10 Tagen werden bereits viele Klone sichtbar. Der Überstand der Primärkulturen wird nach dem in Beispiel 3 beschriebenen ELISA-Verfahren getestet. Primärkulturen, die Antigen-spezifische Antikörper enthalten,werden mit Hilfe eines fluoreszenzaktivierenden Zellsorters auf 96er-well-Zellkulturplatten (Firma Nunc) weiter kloniert. Als Wachstumsfaktor wird dem Medium 5 U/ml IL 6 zugesetzt.

Auf diese Weise konnten beispielsweise die beiden Hybridoma-Zellinien Klon 1.47.74 und Klon 1.17.44 isoliert werden, die bei der Hinterlegungsstelle ECACC (= European Collection of Animal Cell Cultures) unter den Hinterlegungsnummern

1.47.74 ECACC 90071902
1.17.44 ECACC 90071901

hinterlegt worden sind.

Aszitesgewinnung

Zur Erzeugung von Aszites werden 5 x $10^6$ Hybridzellen intraperitoneal Mäusen gespritzt, die zuvor 1-2 mal mit 0,5 ml Pristan vorbehandelt worden sind. Eine bis drei Wochen danach kann aus den Mäusen Aszitesflüssigkeit gewonnen werden. Hieraus können mittels HPCL oder FPLC die Antikörper isoliert werden. Diese monoklonalen Antikörper sind spezifisch gegen Histamin-ITCB gerichtet und zeigen keine bzw. nur eine geringe Kreuzreaktivität mit anderen primären und sekundären Aminen.

Zur Ermittlung der Relativen Affinität werden Kompetitionskurven mit dem homologen Antigen ermittelt und ausgewertet. Die hierfür erforderlichen Messungen werden analog Beispiel 4 durchgeführt.

Beispiel 3

Screening-Test auf Antikörper gegen Histamin-ITCB

Um die Anwesenheit und Spezifität von Antikörpern gegen Histamin-ITCB im Serum immunisierter Mäuse oder im Kulturüberstand der Hybridzellen oder im Aszites zu bestimmen, wird ein kompetetiver ELISA (= enzym linked immunosorbent assay) durchgeführt.

Herstellung von Histamin-ITCB-DADOO-Biotin 1

97 mg (0,25 mmol) Histamin-ITCB-NHS **4** (Fig. 2) werden in 10 ml Dioxan/DMF 5/2 (v/v) gelöst und mit 94 mg (0,25 mmol) N-Biotinyl-1,8-diamino-3,6-dioxaoctan (Biotin-DADOO, Fa. Boehringer Mannheim, Nr. 1112 074) versetzt. Man läßt 16 h bei 20 °C rühren, dann wird das Lösungsmittel im Vakuum entfernt und der ölige Rückstand in ca. 5 ml Essigester/Methanol 50/50 (v/v) gelöst. Man gibt die Lösung auf eine Kieselgelsäule (2,5 x 40 cm) und eluiert mit Essigester/Methanol/Eisessig 50/50/1 (v/v/v). Nachdem die schneller als das Produkt laufenden Verunreinigungen eluiert sind, wechselt man das Laufmittel gegen Essigester/Methanol/Ammoniak 50/50/1 (v/v/v). Das Produkt wird von der Säule gewaschen und die entsprechenden Fraktionen vereinigt. Man dampft den Rückstand im Vakuum ein, löst das verbleibende, zähölige Produkt in 20 ml Wasser und lyophilisiert.

Ausbeute: 68 mg (42 % d.Th.) weißes Lyophilisat.

DC: Kieselgel, Essigester/Methanol/Ammoniak 50/50/1 (v/v/v; Rf = 0,28.

Testablauf:

Mikrotiterplatten werden mit 100 µl pro well einer Lösung aus 10 µg Thermo-SA-Streptavidin/ml (Herstellung nach EP-A 0269 092) in Puffer pH 9,6 eine Stunde bei Raumtemperatur beschichtet. Die Platten werden mit 0,9% Natriumchloridlösung gewaschen. Anschließend wird mit 200 µl pro well einer Lösung von 5 ng/ml, Histamin-ITCB-DADOO-Biotin (Herstellung s.o.) in PBS/1 % RSA (= phosphat buffered saline mit 1 % Rinderserumalbumin) eine Stunde bei Raumtemperatur beschichtet und erneut mit 0,9 % Natriumchloridlösung gewaschen. Darauf werden 100 µl Probe in PBS eine Stunde bei Raumtemperatur inkubiert und erneut mit 0,9 % Natriumchloridlösung gewaschen. Es folgt eine weitere Inkubation (1 Stunde bei Raumtemperatur) mit 100 µl/well einer Lösung von 150 mU/ml eines Schaf-anti-Maus-IgG-Peroxidase-Konjugats (Boehringer Mannheim GmbH, Best.-Nr. 821489) in PBS/O,5 % RSA. Nach einem weiteren Waschschritt mit 0,9 % Natriumchloridlösung wird die Peroxidaseaktivität durch Zugabe von 100 µl/well einer ABTS®-Lösung (100 mg ABTS; 3,25 mmol/l Natriumperborat; 39,8 mmol/l Citronensäure; 60 mmol/l Natriumphosphat; pH 4,4-4,5) 30 min bei Raumtemperatur und anschließender Extinktionsmessung bei 405 nm bestimmt.

| | Messwerte(E) Seren der immunisierten Mäuse | | | | | | |
|---|---|---|---|---|---|---|---|
| Verd. 1: | Maus-Nr. 1 | 2 | 3 | 3 | 5 | 6 | 7 |
| 100 | 1.098 | 1.262 | 1.064 | 1.028 | 1.333 | 1.136 | 0.908 |
| 1000 | 0.736 | 1.135 | 0.801 | 0.955 | 0.973 | 0.835 | 0.874 |
| 10000 | 0.252 | 0.353 | 0.273 | 0.390 | 0.252 | 0.262 | 0.487 |
| 100000 | -0.037 | -0.027 | -0.031 | -0.002 | -0.042 | -0.038 | -0.036 |

Auffallend ist hierbei, daß bei einer Verdünnung von 1:10000 bei allen 7 Mäusen durchweg sehr hohe Messwerte (deutlich über 0.050) erhalten werden, die Titer bei allen Mäusen also sehr hoch sind.

Das primäre Ziel, Antikörper gegen ein ITCB-Derivat eines niedermolekularen Haptens zu erzeugen, ist somit erreicht.

Beispiel 4

Bestimmung der Relativen Affinität und der Kreuzreaktivität verschiedener erfindungsgemäßer monoklonaler Antikörper mit anderen Serumbestandteilen

Es wurde wie in Beispiel 3 beschrieben, vorgegangen. Zunächst wurde die Relative Affinität der Antikörper gegen Histamin-ITCB bestimmt.

Um die Relative Affinität gegen Histamin-ITCB zu bestimmen, wurde den Antikörpern der Analyt Histamin-ITCB in steigenden Konzentrationen zugegeben und die Proben in den unter Beispiel 3 beschriebenen ELISA eingesetzt.

Testprinzip

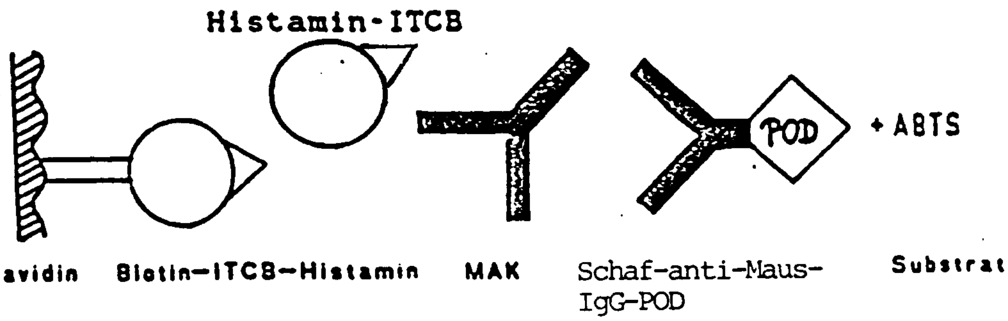


Die Relative Affinität eines Antikörpers ist definiert, als die Menge Histamin-ITCB in ng/ml, die man benötigt, um im vorgegebenen ELISA einen Signalabfall von 50 % zu erhalten. Dieser Signalabfall wird als 50 % Intercept (50 % IC) bezeichnet.

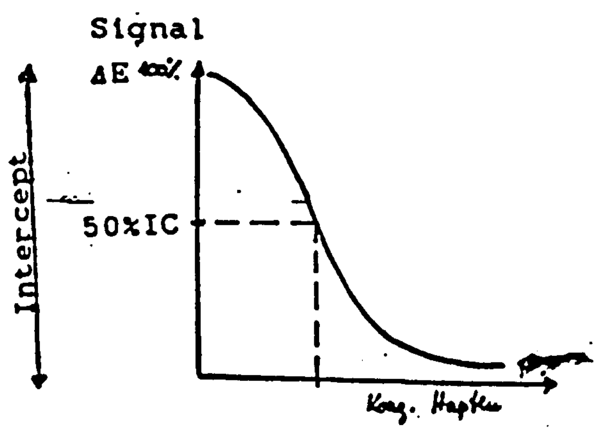


mE (50 % IC) = 1/2 E max ---→ zugeordnete Histamin-ITCB-Konz. entspricht der Relativen Affinität des Antikörpers

Dann wurde zu dem jeweiligen monoklonalen Antikörper jeweils die auf Kreuzreaktiviät zu prüfende Substanz (Histidin, Lysin, Serotonin etc.) in steigender Konzentration zugegeben.

Die Kreuzreaktion wurde anschließend mit folgender Formel berechnet:

$$\frac{C(\text{Histamin-ITCB}) \times 100}{C(\text{kreuzreagierende Substanz})} = \%\ \text{Kreuzreaktion}$$

C = Konzentration des jeweiligen Antigens, die zur Erreichung von 50 % des maximalen Signals erforderlich ist.

In Tabelle 1 sind die gemessenen Werte zusammengestellt.

Tabelle 1: Kreuzreaktion von erfindungsgemäßen monoklonalen Anitkörpern gegen verschiedene Substanzen

| Antikörper | 1.17.44 | 1.25.53 | 1.36.59 | 1.47.74 | 1.54.95 | 1.68.168 | 1.83.201 | 1.97.235 |
|---|---|---|---|---|---|---|---|---|
| | (ECACC 90071901) | | | (ECACC 90071902) | | | | |
| Relative Affinität zu Histamin-ITCB (ng/ml) | 0,5 | 1 | 1 | 0,8 | 1,5 | 1,4 | 0,75 | 1,5 |
| Kreuzreaktion (%) mit: | | | | | | | | |
| Histamin | 0,02 | 0,011 | 0,025 | 0,018 | 0,019 | 0,019 | 0,015 | 0,018 |
| Histidin-ITCB | 0,011 | 0 | 0 | 0 | 0 | 0 | 0 | 0 |
| Lysin-ITCB | 0 | 0 | 0 | 0 | 0 | 0 | 0 | 0 |
| Methylhistamin-ITCB | 0,008 | 0 | 0,009 | 0,001 | 0,002 | 0,004 | 0,001 | 0,001 |
| Acetylhistamin-ITCB | 0,007 | 0,0014 | 0,024 | 0,017 | 0,03 | 0,04 | 0,01 | 0,016 |
| Serotonin-ITCB | 0 | 0 | 0 | 0 | 0 | 0 | 0 | 0 |
| Dopamin-ITCB | 0 | 0 | 0 | 0 | 0 | 0 | 0 | 0 |
| Adrenalin | 0 | 0 | 0 | 0 | 0 | 0 | 0 | 0 |
| Clemastine | 0 | 0 | 0 | 0 | 0 | 0 | 0 | 0 |
| Diphenhydramine | 0 | 0 | 0 | 0 | 0 | 0 | 0 | 0 |
| Cimetidin | 0 | 0 | 0 | 0 | 0 | 0 | 0 | 0 |
| Ranitidin | 0 | 0 | 0 | 0 | 0 | 0 | 0 | 0 |

Beispiel 5

Bestimmung des Analyten Histamin-ITCB in Plasma

Rezeptorlösung:

Aszites von Klon 1.47.74, 1:200 000 verdünnt in PBS

Probe:

Unverdünntes Plasma (1 mg EDTA/ml)), bei - 20 °C gelagert

Eichkurve:

Histamin-ITCB Stammlösung 1 mg/ml Methanol (Verdünnungsreihe in Serum oder Plasma)

A) Probenvorbereitung

Den Probanden werden mit einer großlumigen Kanüle 50 ml Blut in eine Plastikspritze abgenommen und dasselbe sofort in EDTA-beschichtete Röhrchen gegeben und 10 min bei 2600 UPM und +4 °C abzentrifugiert. Das gewonnene Plasma wird in 0,5 ml und 1 ml Aliquots aufgeteilt und bei -20 °C tiefgefroren.

B) Versuchsablauf

Derivatisierung der Proben:

Zunächst wird eine Standardverdünnungsreihe aus Histamin-ITCB (in Serum oder Plasma) angefertigt. Für den Umsetzungansatz werden 14 ml 1 mol/l Natriumbicarbonat-Puffer, pH 9,0 mit 2 ml ITCB Lösung (61 mg ITCB/ml in DMSO) gemischt. Jeweils 280 µl dieses ITCB-Puffergemischs und 80 µl Standard bzw. Probe werden 30 sec. geschüttelt und eine Stunde bei 56 °C im Wasserbad inkubiert. Um die Reaktion abzustoppen werden 40 µl 1,8 mol/l Tris-Puffer pH 4,1 zupipettiert und es wird erneut geschüttelt.

Einsatz der Proben im ELISA-Test:

Mit einer 12-Kanal-Pipette werden jeweils 50 µl des verdünnten Aszites (Antikörper) und 50 µl der Probe in jede Vertiefung einer, nach Beispiel 2, beschichteten Mikrotiterplatte gegeben und für eine Stunde bei Raumtemperatur, unter Schütteln, inkubiert. Nach zweimaligem Waschen mit 0,9 % Natriumchloridlösung wird mit 100 µl Schaf-anti-Maus-POD-Konjugat (s. Beispiel 3) beladen und eine Stunde bei Raumtemperatur inkubiert. Nach dreimaligem Waschen mit Waschpuffer wird mit 100 µl ABTS-Substratlösung beladen und nach 30 min. Farbreaktion die Extinktion bei 405 nm in einem ELISA-Reader (Dynatec) gemessen.

| | |
|---|---|
| Meßbereich : | Proben von 750 pg - 100 ng Histamin/ml Probenverdünnung (1:10) |
| Empfindlichkeit des Tests : | 75 pg-10 ng/ml |

Beispiel 6

Histamin-Freisetzung aus Basophilen Leukozyten

Nach der im Photometrischen Allergie-Degranulationstest (PAD-Test; Boehringer Mannheim GmbH, Best.-Nr. 854506) beschriebenen Methode werden Leukozyten aus dem Vollblut angereichert und mit einem Allergen inkubiert. Die bei der Antigen-/Antikörperreaktion freigesetzte Histaminmenge wird nach unten beschriebenen Methode (Derivatisierung mit ITCB--> kompetetiver ELISA) im Überstand gemessen.

Leukozytengewinnung:

20 ml venöses Vollblut wird mit einer großlumigen Kanüle in EDTA-beschichtete Rörchen (2 ml EDTA-Lösung) abgenommen und in einem Sarstedt Serum-Plasma-Filter (Best. Nr.53.422; 25 ml) gegeben, in

dem ein Gemisch aus :
4 ml Glucoselösung (6 %) (Lösung 8)
4 ml Dextran (6 %) (Lösung 2)
0,8 ml Gelatine (2 %) (Lösung 7)
vorgelegt wird.

Nach vorsichtigem Mischen erfolgt die Sedimentation der Erytrozyten innerhalb von 60-90 min bei Raumtemperatur. Der Plasmaüberstand (ca. 10 ml) wird möglichst vollständig abgenommen und gleichmäßig in zwei Plastik-Zentrifugenröhrchen (Sarstedt 55.468; 13 ml) aufgeteilt. Den Röhrchen werden jeweils 5 ml TRIS-Puffer (Lösung 9) zugesetzt und anschliessend 15 min bei 400 - 500 g zentrifugiert. Der Überstand wird abdekantiert, die Zellen mit 10 ml TRIS-Puffer gewaschen und wieder 15 min zentrifugiert.

Nach Abdekantieren des Überstandes werden die Zellen in insgesamt 12 ml (2 x 6 ml) eiskaltem TRIS-Puffer/Glucose (Lösung 10) resuspendiert und zur Degranulation eingesetzt.

Zur Bestimmung der Zellzahl werden 50 µl Zellsuspension mit 450 µl 3 %iger Essigsäure verdünnt und in einer Thoma-Zählkammer ausgezählt.

Inkubation mit Antigen (Degranulation)

In Eppendorf-Reaktionsgefäße pipettieren:

| Puffer | Probenleerwert | Probe | Anti-IgE/ Allergen Leerwert | Leerwert |
|---|---|---|---|---|
| TRIS-Puffer/Glucose(37°C)(10) | 130 µl | 130 µl | 130 µl | 130 µl |
| Leukozyten-Suspension | 300 µl | 300 µl | -- | -- |
| TRIS-Puffer/Glucose(37°C)(10) | -- | -- | 300 µl | 300 µl |
| 5 min bei 37°C inkubieren | | | | |
| Allergenlösung (12) | -- | 30 µl | 30 µl | -- |
| TRIS-Puffer/Glucose (10) | 30 µl | -- | -- | 30 µl |
| 1 min bei 37°C inkubieren | | | | |
| Starter-Lösung ($CaCl_2$) (4) | 30 µl | 30 µl | 30 µl | 30 µl |
| vorsichtig mischen, 60 min bei 37°C inkubieren | | | | |
| Stopper-Lösung (eiskalt) (3) | 170 µl | 170 µl | 170 µl | 170 µl |

Die Proben wurden nach der Histamin-Freisetzung 5 min in Eppendorf-Zentrifuge bei 900 g und 4 °C zentrifugiert. Die Überstände wurden innerhalb von 15 min vom Zellrückstand abgetrennt und bei 4 °C oder tiefgefroren (-20 °C) bis zur Histaminbestimmung aufbewahrt.

Lösungen

A.Stammlösungen

(1) TRIS-Konzentrat (225 mmol/l)
27,2 g TRIS, 69,0 g NaCl und 3,7 g KCl in 1 l Aqua bidest. lösen und mit konz. HCl auf pH 9,6 einstellen.
(2) Dextran
Dextranlösung:
60 g Dextran (MW 75.000) in 11 0,9 % NaCl lösen.
(3) EDTA (100 mmol/l)
3,72 g EDTA in 100 ml 0,9 % NaCl lösen und mit NaOH auf pH 7,6 einstellen.
(4) Start-Lösung($CaCl_2$):
1,82 g $CaCl_2$ x 2 $H_2O$ in 100 ml 0,9 % NaCl lösen.
(5) TRIS/HCl-Puffer TN (0,075 mol/l; pH 7,6)
Stammlösung a:
36,354 g TRIS in 1 l Aqua bidest. lösen.
Stammlösung b:
0,1 mol/l HCl
Puffer: 25 ml Stammlösung a
\+ 58,95 ml Stammlösung b
(6) NaCl (0,9 %)
B.Gebrauchslösungen
(7) Gelatine (2 %)
2,0 g Gelatine in 100 ml 0,9 % NaCl unter Erwärmen (max. 50 °C) lösen.
(8) Glucose (6%)
6,0 g $\alpha$-D-Glucose, wasserfrei in 100 ml 0,9 % NaCl lösen.
(9) TRIS-Puffer (22,5 mmol/l)
50 ml TRIS-Konzentrat (Lösung 1)
400 ml $H_2O$ bidest.
12,4 ml Gelatinelösung (Lösung 7)
5,0 ml EDTA-Lösung (Lösung 3)
mit Aqua bidest. auf 500 ml auffüllen, pH (7,6) überprüfen und ggf. nachstellen.
(10) TRIS-Puffer/Glucose
2,7 ml Glucoselösung (Lösung 8) mit 150 ml TRIS-Puffer (Lösung 9) mischen.
(11) Antiserum gegen Human-IgE (1:500)
Fa. Behring (OTNP 04/05) z. B. 464748 IU/ml
10 µl Antiserum mit 5000 µl TRIS-Puffer/Glucose (Lösung 12) verdünnen.
(12) Allergen-Extrakt
Allergenkonzentrationen von 0,1/1/10 µg/ml durch Verdünnen mit TRIS-Puffer/Glucose (Lösung 10) herstellen.

Beispiel 7

Histaminfreisetzung aus Vollblut

Nach der von Siraganian (Manual of Clinical Immunology, 1976 Chapter 80, 602) beschriebenen Methode wird Vollblut mit dem Antigen inkubiert und anschließend zentrifugiert. Die bei der Antigen-/Antikörperreaktion freigesetzte Histaminmenge wird nach der beschriebenen Methode:
Derivatisierung mit ITCB und anschließendem kompetetivem ELISA im Überstand gemessen.

Probengewinnung

20 ml venöses Vollblut wird mit einer Plastikspritze entnommen und in Plastikröhrchen (Falcon, 50 ml) mit 0,5 ml Heparin (Liquemin sodium 10, 1ml = 1,000 U;Organo Inc., West Orange, N.J.) gegeben. Bis zum Einsatz in den Test kann das Blut bis zu 24 Stunden bei 4 °C im Kühlschrank gelagert werden. Das Blut wird mit 5 ml PIPES ACM (Lösung 7) verdünnt und direkt in den Test eingesetzt.

Inkubation mit Antigen (Histaminfreisetzungsreaktion)

Die Reaktion wird in 75 mm Plastikröhrchen (Falcon 2052) durchgeführt.

| | Probenleerwert | Probe | Totalhistamin der Probe |
|---|---|---|---|
| PIPES ACM (7) | 500 µl | -- | -- im Eisbad |
| Allergenlösung (8) | -- | 500 µl | -- |
| Verdünntes Vollblut | 500 µl | 500 µl | 500 µl |
| 12 % Perchlorsäure(4) | -- | -- | 500 µl |
| Röhrchen in 37 °C-Wasserbad überführen 1 h inkubieren (alle 10 min schütteln) | | | |
| Stoppen der Reaktion: Röhrchen in Eisbad überführen<br>Zentrifugation: 30 min bei 2,500 UPM (1,200 x g), 4 °C. | | | |

Den Überstand bis zur Histaminbestimmung bei -20 °C einfrieren.

Lösungen:

A. Stammlösungen:

(1) PIPES-Puffer (10 fach konzentriert)
75,60 g PIPES
69,28 g NaCl
3,72 g KCl
43 ml 10 N NaOH
in 1 l Aqua bidest. lösen. (pH 7.48)
(2) Humanserumalbumin-Lösung (25 %)
(3) EDTA-Lösung (0,1 mol/l)
37,23 g EDTA in 600 ml Aqua bidest. lösen und mit 50 % NaOH pH 7,18 - 7,20 einstellen und mit Aqua bidest. auf 1 l auffüllen.
(4) Perchlorsäure (12%)
200 ml 60 % $HClO_4$ mit 800 ml Aqua bidest. verdünnen.
(5) $Ca^{2+}$-Lösung (0,1 mol/l)
(6) $Mg^{2+}$-Lösung (0,1 mol/l)

B. Gebrauchslösungen

(7) PIPES ACM-Puffer (1 mmol/l $Ca^{2+}$, 0,5 mmol/l $Mg^{2+}$)
PIPES A-Puffer:
10 ml PIPES-Stammlösung (10 fach konz.) werden mit Aqua bidest. auf 100 ml verdünnt und 0,125 ml der 25 % Humanserumalbumin-Lösung dazugegeben.
1 ml 0,1 mol/l $Ca^{2+}$-Lösung
0,5 ml 0,1 mol/l $Mg^{2+}$-Lösung werden mit PIPES A-Puffer auf 100 ml verdünnt.
(8) Allergenlösung
Allergenverdünnungen in PIPES ACM-Puffer ansetzen und aliquotiert bei -20 °C lagern.

Beispiel 8

Synthese von Nitro-ITCB derivatisiertem Adrenalin (siehe auch Figur 3)

**a) 5-Isothiocyanato-2-nitro-benzoesäure 12**

1,82 g (10 mmol) 5-Amino-2-nitrobenzoesäure **11** werden in einem Gemisch aus 10 ml konz. Salzsäure und 80 ml Wasser durch Erwärmen auf 100 ° C gelöst. Nach dem Abkühlen der Lösung auf 20 ° C tropft man 1,37 g (12 mmol) Thiophosgen zu. Der sich nach einer kurzen Induktionsphase bildende Niederschlag wird nach 6 h abgesaugt, mit Wasser gewaschen und im Exsiccator über Calciumchlorid getrocknet.

Ausbeute: 0,78 g gelbes Pulver (44 % d.Th.).
IR (KBr):$\nu$ = 1984, 1964 $cm^{-1}$.
DC: Kieselgel, Essigester/Eisessig 99/1 (v/v); $R_f$ = 0,59.

**b) N'-(3-Carboxy-4-nitrophenyl)-N[2-(3,4-dihydroxyphenyl)-2-hydroxethyl]-N-methylthioharnstoff 13 (Adrenalin-Nitro-ITCB)**

1,83 g (10 mmol) L-Adrenalin **6b** und 2,24 g (10 mmol) 5-Isothiocyanato-2-nitrobenzoesäure **12** werden in 50 ml absolutem THF gelöst und 20 h bei 20 ° C gerührt. Anschließend entfernt man das Lösungsmittel im Wasserstrahlvakuum, nimmt den Rückstand in 25 ml Methanol/Essigester 1/1 (v/v) auf und reinigt das Rohprodukt duch Chromatographie an einer Kieselgelsäule (5 x 5 cm). Als Eluent dient Methanol/Essigester 1/1 (v/v). Die das Produkt **13** enthaltenden Fraktionen werden vereinigt und auf 100 ml eingeengt. Dann versetzt man die Lösung mit 200 mg Aktivkohle und rührt 16 h bei 20 ° C. Die Aktivkohle wird abfiltriert, das Lösungsmittel im Wasserstrahlvakuum entfernt und der Rückstand an der Hochvakuum-Pumpe getrocknet.

Ausbeute: 3,45 g gelber Feststoff (85 % d.Th.)
DC: Kieselgel, Essigester/Eisessig 99/1 (v/v); $R_f$ : = 0,27.

**c) N-[2-(3,4-Dihydroxyphenyl)-2-hydroxyethyl]-N-methyl-N'-[4-nitro-3-(N-succinimidyloxycarbonyl)-phenyl]-thioharnstoff 14 (Adrenalin-Nitro-ITCB-NHS)**

2,03 g ( 5 mmol) Adrenalin-Nitro-ITCB **13** werden in 50 ml absolutem Dioxan gelöst und unter Rühren mit 0,61 g (5,3 mmol) N-Hydroxysuccinimid und 1,24 g (6 mmol) Dicyclohexylcarbodiimid versetzt. Nach 20 h Rühren bei 20 ° C wird der entstandene Dicyclohexylharnstoff abfiltriert und das Lösungsmittel im Wasserstrahlvakuum bei 50 ° C entfernt. Der Rückstand wird nochmals in 20 ml Dioxan aufgenommen und Unlösliches abfiltriert. Anschließend dampft man zur Trockene ein und reinigt das Rohprodukt mittels Säulenchromatographie an Kieselgel (5 x 5 cm). Als Eluent dient Essigester. Die das Produkt **14** enthaltenden Fraktionen werden auf 10 ml eingeengt und langsam unter Rühren zu 100 ml Diisopropylether getropft. Der sich bildende Niederschlag wird abgetrennt und an der Hochvakuum-Pumpe getrocknet.

Aubeute: 0,6 g gelber Feststoff (24 % d.Th.)

d) Adrenalin-Nitro-ITCB-$\beta$-Galactosidase-Immunogen **15**

Die Herstellung erfolgt analog **5** (Beispiel 1c).

**Patentansprüche**

1. Verfahren zur Gewinnung eines monoklonalen Antikörpers gegen ein primäres oder sekundäres biogenes Amin, wobei der Antikörper gegen ein Kopplungsprodukt aus dem biogenen Amin und einem Kopplungsreagenz gerichtet ist,
**dadurch gekennzeichnet,**
daß man
(1) ein primäres oder sekundäres biogenes Amin über seine Aminofunktion an ein Kopplungsreagenz bindet, welches die allgemeine chemische Struktur besitzt:

X = C = N-R-Y

worin

X für O oder S steht,
R eine nicht substituierte oder substituierte aromatische, heteroaromatische oder Cycloalkylgruppe ist,
Y für eine Carboxylgruppe mit der allgemeinen Formel

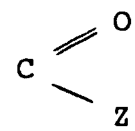

steht, worin Z (a) OH oder $O^-$ oder (b) ein anderer unter den Bedingungen der Kopplungsreaktion nicht mit einer Aminofunktion reagierender Rest $Z^1$ ist, der jedoch durch einen oder mehrere Reaktionsschritte in einen reaktiven Ester-, Säurechlorid- oder Säureanhydridrest überführbar ist,
(2) den Rest Z der Carboxylgruppe des in Schritt (1) entstehenden Kopplungsprodukts in einen reaktiven Ester-, Säurechlorid- oder Säureanhydridrest überführt,
(3) das aktivierte Kopplungsprodukt aus Schritt (2) an ein Trägermaterial bindet, so daß man ein Trägergebundenes Kopplungsprodukt erhält, das als Immunogen dient,
(4) ein Versuchstier mit dem Immunogen aus Schritt (3) immunisiert, und
(5) auf an sich bekannte Weise monoklonale Antikörper gegen das Kopplungsprodukt aus dem biogenen Amin und dem Kopplungsreagenz aus dem immunisierten Versuchstier gewinnt.

2. Verfahren nach Anspruch 1,
**dadurch gekennzeichnet,**
daß man 3-Isothiocyanatobenzoesäure oder ein Salz oder Derivat davon als Kopplungsreagenz verwendet.

3. Verfahren nach einem der vorhergehenden Ansprüche,
**dadurch gekennzeichnet,**
daß man als biogenes Amin Histamin oder ein Catecholamin, ausgewählt aus Adrenalin, Noradrenalin und Dopamin verwendet.

4. Kopplungsprodukt aus einem primären oder sekundären biogenen Amin $HNR^4R^5$ und einem Kopplungsreagenz, wobei das Kopplungsprodukt folgende allgemeine Struktur besitzt:

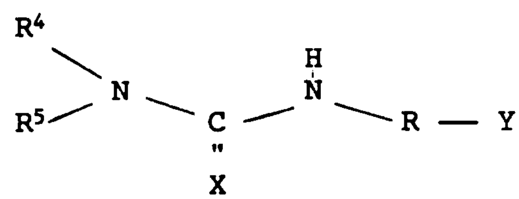

worin $R^4$ und $R^5$ die dem jeweiligen biogenen Amin entsprechenden Reste bedeuten, und
X für O oder S steht,
R eine nicht substituierte oder substituierte aromatische, heteroaromatische oder Cycloalkylgruppe ist,
Y für eine Carboxylgruppe mit der allgemeinen Formel

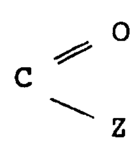

steht, worin Z (a) OH oder $O^-$ oder (b) ein anderer unter den Bedingungen der Kopplungsreaktion nicht mit einer Aminofunktion reagierender Rest $Z^1$ ist, der jedoch in einen reaktiven Ester-, Säurechlorid-

oder Säureanhydridrest überführbar ist.

5. Kopplungsprodukt nach Anspruch 4, **dadurch gekennzeichnet,** daß das biogene Amin $HNR^4R^5$ Histamin oder ein Catecholamin, ausgewählt aus Adrenalin, Noradrenalin und Dopamin ist.

6. Kopplungsprodukt nach Anspruch 4 oder 5,
**dadurch gekennzeichnet,**
daß das Kopplungsreagenz 3-Isothiocyanatobenzoesäure, ein Salz oder Derivat davon ist.

7. Monoklonaler Antikörper gegen ein Kopplungsprodukt nach einem der Ansprüche 4 bis 6 aus einem primären oder sekundären biogenen Amin und einem Kopplungsreagenz.

8. Monoklonaler Antikörper gegen ein Kopplungsprodukt aus Histamin und Isothiocyanatobenzoesäure, sekretiert von der Hybridoma-Zellinie 1.47.74 (ECACC 90071902).

9. Monoklonaler Antikörper gegen ein Kopplungsprodukt aus Histamin und Isothiocyanatobenzoesäure, sekretiert von der Hybridoma-Zellinie 1.17.44 (ECACC 90071901).

10. Verfahren zur Bestimmung eines primären oder sekundären biogenen Amins in einer Probelösung,
**dadurch gekennzeichnet,**
daß man
(1) das biogene Amin in der Probelösung über seine Aminofunktion an ein Kopplungsreagenz bindet, welches die allgemeine chemische Struktur besitzt:

X = C = N-R-Y

worin X für O oder S steht,
R eine nicht substituierte oder substituierte aromatische, heteroaromatische oder Cycloalkylgruppe ist,
Y für eine Carboxylgruppe mit der allgemeinen Formel

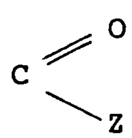

steht, worin Z (a) OH oder $O^-$ oder (b) ein anderer unter den Bedingungen der Kopplungsreaktion nicht mit einer Aminofunktion reagierender Rest $Z^1$ ist, der jedoch in einen reaktiven Ester-, Säurechlorid- oder Säureanhydridrest überführbar ist,
(2) die Probelösung mit einem oder mehreren monoklonalen Antikörpern gegen das in Schritt (1) entstehende Kopplungsprodukt aus biogenem Amin und Kopplungsreagenz inkubiert,
(3) durch Quantifizierung der Antikörper/Antigen-Reaktion auf übliche Weise die Konzentration des biogenen Amins in der Probe bestimmt.

11. Verfahren nach Anspruch 10, **dadurch gekennzeichnet,** daß man als Kopplungsreagenz 3-Isothiocyanatobenzoesäure, ein Salz oder ein Derivat davon verwendet.

12. Verfahren nach Anspruch 10 oder 11,
**dadurch gekennzeichnet,**
daß man als zu bestimmendes biogenes Amin Histamin oder ein Catecholamin, ausgewählt aus Adrenalin, Noradrenalin und Dopamin verwendet.

## Claims

1. Process for the production of a monoclonal antibody against a primary or secondary biogenic amine in which the antibody is directed against a coupling product of the biogenic amine and a coupling reagent,
**wherein**

(1) a primary or secondary biogenic amine is bound via its amino group to a coupling reagent which has the general chemical structure:

X = C = N-R-Y

in which
X represents O or S,
R is a non-substituted or substituted aromatic, heteroaromatic or cycloalkyl group;
Y represents a carboxyl group having the general formula

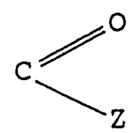

in which Z is (a) OH or $O^-$ or (b) another residue $Z^1$ which does not react with an amino group under the conditions of the coupling reaction but which can be converted by one or several reaction steps into a reactive ester, acid chloride or acid anhydride residue,
(2) the residue Z converts the carboxyl group of the coupling product which is formed in step (1) into a reactive ester, acid chloride or acid anhydride residue,
(3) the activated coupling product from step (2) is bound to a carrier material so that a carrier-bound coupling product is obtained which serves as an immunogen,
(4) a laboratory animal is immunized with the immunogen from step (3) and
(5) monoclonal antibodies against the coupling product of the biogenic amine and the coupling reagent are obtained in a known way from the immunized laboratory animal.

2. Process as claimed in claim 1, **wherein** 3-isothiocyanatobenzoic acid or a salt or derivative thereof is used as the coupling reagent.

3. Process as claimed in one of the previous claims, **wherein** histamine or a catecholamine selected from adrenaline, noradrenaline or dopamine is used as the biogenic amine.

4. Coupling product of a primary or secondary biogenic amine $HNR^4R^5$ and a coupling reagent in which the coupling product has the following general structure:

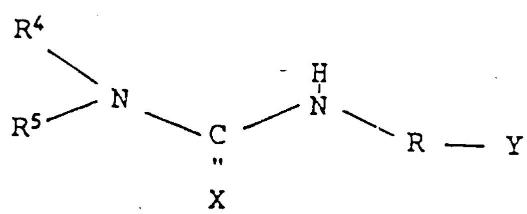

in which $R^4$ and $R^5$ denote residues corresponding to the respective biogenic amine and
X represents O or S,
R is a non-substituted or substituted aromatic, heteroaromatic or cycloalkyl group,
Y represents a carboxyl group having the general formula

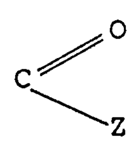

in which Z is (a) OH or $O^-$ or (b) another residue $Z^1$ which does not react with an amino group under the conditions of the coupling reaction but which can be converted into a reactive ester, acid chloride or acid anhydride residue.

**5.** Coupling product as claimed in claim 4,
**wherein** the biogenic amine $HNR^4R^5$ is histamine or a catecholamine selected from adrenaline, noradrenaline or dopamine.

**6.** Coupling product as claimed in claim 4 or 5,
**wherein** the coupling reagent is 3-isothiocyanatobenzoic acid, a salt or derivative thereof.

**7.** Monoclonal antibody against a coupling product as claimed in one of the claims 4 to 6 of a primary or secondary biogenic amine and a coupling reagent.

**8.** Monoclonal antibody against a coupling product of histamine and isothiocyanatobenzoic acid secreted by the hybridoma cell line 1.47.74 (ECACC 90071902).

**9.** Monoclonal antibody against a coupling product of histamine and isothiocyanatobenzoic acid secreted by the hybridoma cell line 1.17.44 (ECACC 90071901).

**10.** Method for the determination of a primary or secondary biogenic amine in a sample solution,
**wherein**

(1) the biogenic amine in the sample solution is bound via its amino group to a coupling reagent which has the general chemical structure:

X = C = N-R-Y

in which
X represents O or S,
R is a non-substituted or substituted aromatic, heteroaromatic or cycloalkyl group;
Y represents a carboxyl group having the general formula

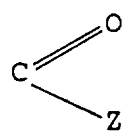

in which Z is (a) OH or $O^-$ or (b) another residue $Z^1$ which does not react with an amino group under the conditions of the coupling reaction but which can be converted into a reactive ester, acid chloride or acid anhydride residue,
(2) the sample solution is incubated with one or several monoclonal antibodies against the coupling product of biogenic amine and coupling reagent which is formed in step (1),
(3) the concentration of the biogenic amine in the sample is determined in the usual way by quantifying the antibody/antigen reaction.

**11.** Method as claimed in claim 10, **wherein** 3-isothiocyanatobenzoic acid, a salt or derivative thereof is used as the coupling reagent.

**12.** Method as claimed in claim 10 or 11,
**wherein** histamine or a catecholamine selected from adrenaline, noradrenaline or dopamine is used as the biogenic amine to be determined.

## Revendications

**1.** Procédé d'obtention d'un anticorps monoclonal contre une amine biogène primaire ou secondaire, dans lequel l'anticorps est dirigé contre un produit de couplage constitué par l'amine biogène et un réactif de couplage,

caractérisé en ce que

(1) on lie une amine biogène primaire ou secondaire par l'intermédiaire de sa fonction amino à un réactif de couplage qui possède la structure chimique générale:

X = C = N-R-Y

dans laquelle
X représente O ou S,
R est un groupe aromatique, hétéroaromatique ou cycloalkyle non substitué ou substitué,
Y représente un groupe carboxyle de formule générale

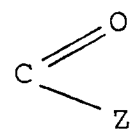

dans laquelle Z est (a) OH ou $O^-$ ou (b) un autre reste $Z^1$ qui ne réagit pas avec une fonction amino dans les conditions de la réaction de couplage, mais qui peut être converti par une ou plusieurs étapes réactionnelles en un reste ester, chlorure d'acide ou anhydride d'acide réactif,

(2) on convertit Le reste Z du groupe carboxyle du produit de couplage formé dans l'étape (1) en un reste ester, chlorure d'acide ou annydride d'acide réactif,

(3) on lie le produit de couplage activé de l'étape (2) à un matériau support de sorte que l'on obtient un produit de couplage lié à un support qui sert d'immunogène,

(4) on immunise un animal d'expérience avec l'immunogène de l'étape (3), et

(5) on obtient de manière connue en soi à partir de l'animal d'expérience immunisé des anticorps monoclonaux contre le produit de couplage constitué par l'amine biogène et le réactif de couplage.

**2.** Procédé selon la revendication 1, caractérisé en ce que l'on utilise comme réactif de couplage l'acide 3-isothiocyanatobenzoïque ou un sel ou dérivé de celui-ci.

**3.** Procédé selon l'une des revendications précédentes, caractérisé en ce que l'on utilise comme amine biogène l'histamine ou une catécholamine choisie parmi l'adrénaline, la noradrénaline et la dopamine.

**4.** Produit de couplage constitué par une amine biogène primaire ou secondaire $HNR^4R^5$ et par un réactif de couplage, le produit de couplage possédant la structure générale suivante:

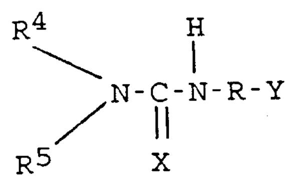

dans laquelle $R^4$ et $R^5$ représentent les restes correspondant à l'amine biogène en question, et
X représente O ou S,
R est un groupe aromatique, hétéroaromatique ou cycloalkyle non substitué ou substitué,
Y représente un groupe carboxyle de formule générale

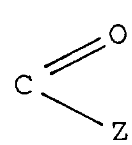

dans laquelle Z est (a) OH ou $O^-$ ou (b) un autre reste $Z^1$ qui ne réagit pas avec une fonction amino dans les conditions de la réaction de couplage, mais qui peut être converti en un reste ester, chlorure d'acide ou anhydride d'acide réactif.

**5.** Produit de couplage selon la revendication 4, caractérisé en ce que l'amine biogène $HNR^4R^5$ est l'histamine ou une catécholamine choisie parmi l'adrénaline, la noradrénaline et la dopamine.

**6.** Produit de couplage selon la revendication 4 ou 5, caractérisé en ce que le réactif de couplage est l'acide 3-isothiocyanatobenzoïque ou un sel ou dérivé de celui-ci.

**7.** Anticorps monoclonal contre un produit de couplage selon l'une des revendications 4 à 6 constitué par une amine biogène primaire ou secondaire et par un réactif de couplage.

**8.** Anticorps monoclonal contre un produit de couplage constitué par l'histamine et l'acide isothiocyanatobenzoïque, sécrété par la lignée cellulaire d'hybridome 1.47.74 (ECACC 90071902).

**9.** Anticorps monoclonal contre un produit de couplage constitué par l'histamine et l'acide isothiocyanatobenzoïque, sécrété par la lignée cellulaire d'hybridome 1.17.44 (ECACC 90071901).

**10.** Procédé de détermination d'une amine biogène primaire ou secondaire dans une solution échantillon, caractérisé en ce que

(1) on lie l'amine biogène primaire ou secondaire dans la solution échantillon par l'intermédiaire de sa fonction amino à un réactif de couplage qui possède la structure chimique générale:

X = C = N-R-Y

dans laquelle
X représente O ou S,
R est un groupe aromatique, hétéroaromatique ou cycloalkyle non substitué ou substitué,
Y représente un groupe carboxyle de formule générale

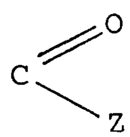

dans laquelle Z est (a) OH ou $O^-$ ou (b) un autre reste $Z^1$ qui ne réagit pas avec une fonction amino dans les conditions de la réaction de couplage, mais qui peut être converti en un reste ester, chlorure d'acide ou anhydride d'acide réactif,

(2) on incube la solution échantillon avec un ou plusieurs anticorps monoclonaux contre le produit de couplage formé dans l'étape (1), constitué par l'amine biogène et le réaction de couplage,

(3) on détermine la concentration de l'amine biogène dans l'échantillon par quantification de la réaction antigène/anticorps de manière habituelle.

**11.** Procédé selon la revendication 10, caractérisé en ce que l'on utilise comme réactif de couplage l'acide 3-isothiocyanatobenzoïque ou un sel ou dérivé de celui-ci.

**12.** Procédé selon la revendication 10 ou 11, caractérisé en ce que l'on utilise comme amine biogène à déterminer l'histamine ou une catécholamine choisie parmi l'adrénaline, la noradrénaline et la dopamine.

Fig. 1

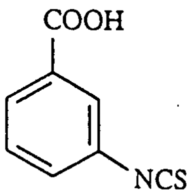

1

1 +

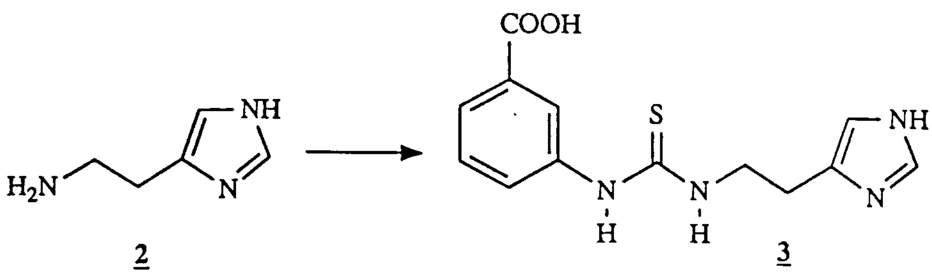

2

3

3 +

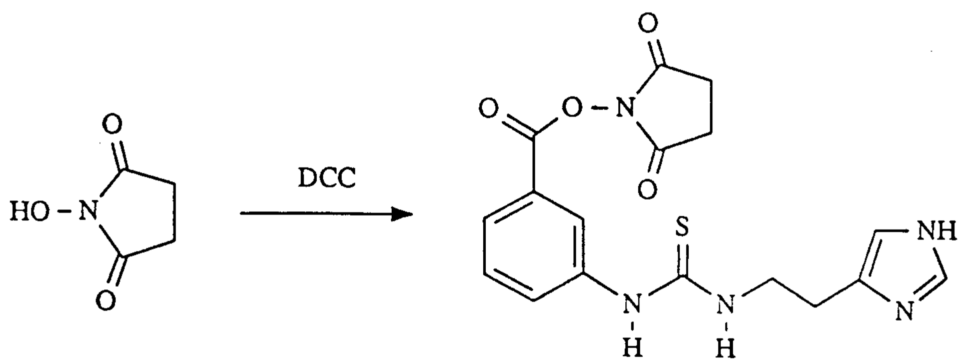


4

4 + $H_2N$-ß-Gal

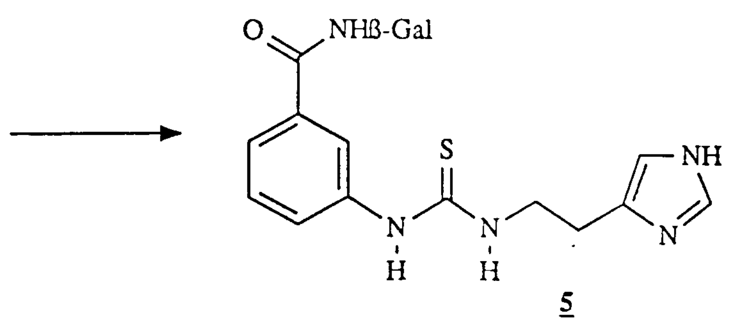

5

Fig. 2

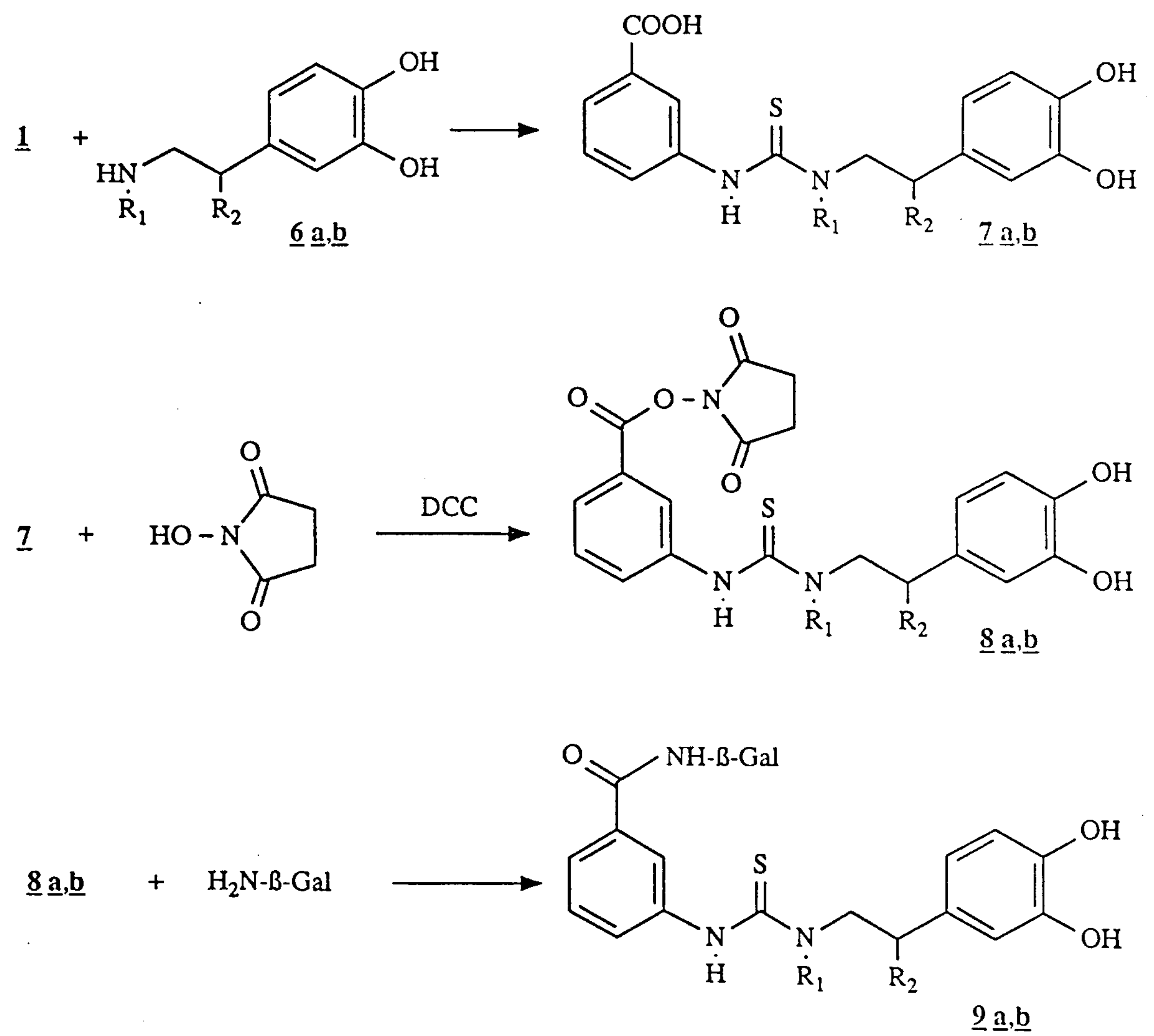


1 + 6 a,b → 7 a,b

7 + → 8 a,b

8 a,b + $H_2N$-ß-Gal → 9 a,b

6 a : $R_1 = H$ $R_2 = H$ (Dopamin)

6 b : $R_1 = CH_3$ $R_2 = OH$ (Adrenalin)

Fig.3

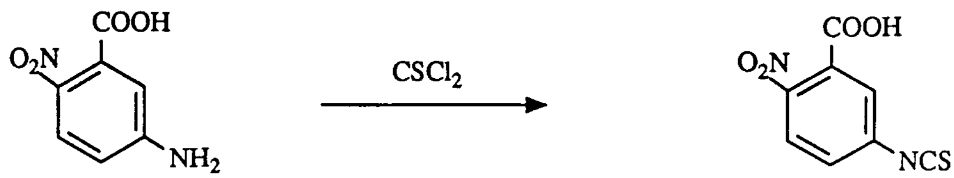


11 12

12 + 6 b

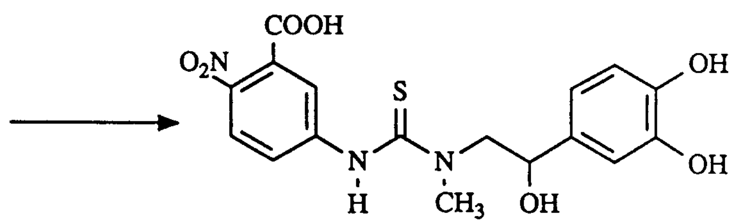

13

13 +

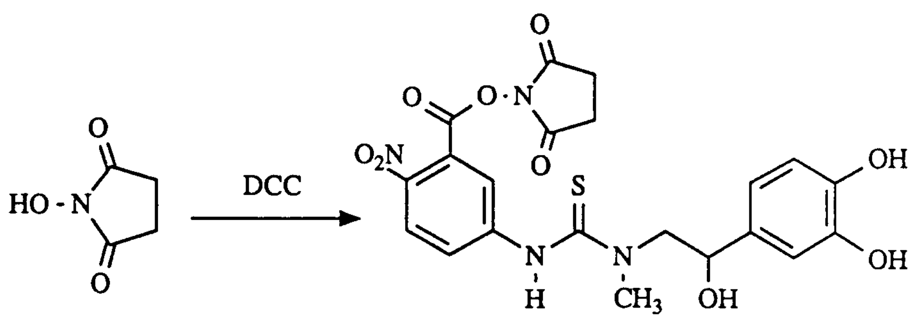


14

14 + $H_2N$-ß-Gal

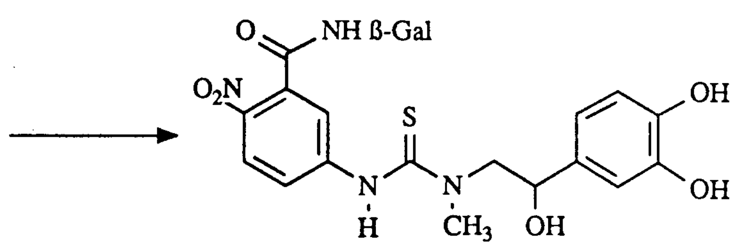

15